# EUROPEAN PATENT SPECIFICATION

(11) **EP 2 101 836 B1**
(45) Date of publication and mention of the grant of the patent: **26.11.2014**
(21) Application number: 07855068.8
(22) Date of filing: 11.12.2007
(51) Int. Cl.: A61L 27/54, A61L 27/56, A61L 29/14, A61L 29/16, A61L 31/14, A61L 31/16, A61M 25/00, A61M 31/00, A61F 2/00

(54) **IMPLANTABLE MEDICAL DEVICE WITH PHARMOCOLOGICALLY ACTIVE INGREDIENT**
IMPLANTIERBARES MEDIZINPRODUKT MIT EINEM PHARMAKOLOGISCHEN WIRKSTOFF.
DISPOSITIF MÉDICAL IMPLANTABLE CONTENANT UNE SUBSTANCE PHARMACEUTIQUEMENT ACTIVE

(30) Priority: 13.12.2006 US 638141
(43) Date of publication of application: 23.09.2009
(73) Proprietor: Cook Medical Technologies LLC, Bloomington, IN 47404 (US)
(72) Inventor: MILLER, Jessica Watts, Terre Haute, IN 47803 (US); FISCHER, Frank J., Bloomington, IN 47401 (US)
(74) Representative: Garratt, Peter Douglas
(86) International application number: PCT/US2007/087064
(87) International publication number: WO 2008/076706

(56) References cited:
- WO-A-90/06094
- WO-A-2004/091444
- WO-A-2006/121969
- DE-A1- 10 024 752
- US-A- 5 902 283
- US-B1- 6 599 275

## Description

### Technical Field

This invention relates generally to medical devices and, particularly, to medical devices that are implantable either partly or completely into a human or veterinary patient.

### Background of the Invention

It has become common to treat a variety of medical conditions by introducing an implantable medical device partly or completely into the esophagus, trachea, colon, biliary tract, urinary tract, vascular system or other location with a human or veterinary patient. For example, many treatments of the vascular system entail the introduction of a device such as a stent, a catheter, a balloon, a wire guide, a cannula, or the like. However, when such a device is introduced into and manipulated through the vascular system, the blood vessel walls can be disturbed or injured. Clot formation or thrombosis often results at the injured site, causing stenosis or occlusion of the blood vessel. Moreover, if the medical device is left within the patient for an extended period of time, a thrombus often forms on the device itself, again causing stenosis or occlusion. As a result, the patient is placed at risk of a variety of complications, including heart attack, pulmonary embolism, and stroke. Thus, the use of such a medical device can entail the risk of precisely the problems that its use was intended to ameliorate.

Another problem associated with implantable medical devices and, more particularly, to partly implanted medical devices such as catheters percutaneously introduced into the vascular system of a patient for long-term hemodialysis or drug infusion is the risk of infection. This risk is also present with hyperalimentation (intravenous feeding) catheters which are percutaneously introduced into the patient. In addition, similar risks exist in the urinary tract of the patient when an urethral catheter, such as a well-known Foley catheter, is introduced into the patient's bladder via the urethra for the drainage of urine.

An attempt to reduce the risk of infection is to use a pharmacologically active ingredient, such as an antibiotic, in conjunction with the catheter. Various coatings including antibiotics have been utilized in the past; however, the antibiotic typically is dispersed or dissipated from the coating in a relatively short period of time. Although effective in short-term implantation, such coatings are typically ineffective for extended duration placement such as with hemodialysis, drug infusion, or urinary tract catheters, which can be implanted in the patient for two to three years at a time.

Reference is directed to US 6 599 275 which discloses a catheter with inner and outer tubes and an intermediate later of silicone with a pharmacologically active ingredient mixed therein.

### Summary of the Invention

The foregoing problems are solved and a technical advance is achieved in an improvement to a medical device that is implantable either partly or completely into a human or veterinary patient. The present invention is set forth in the appended claims. There is here described an implantable medical device having an outer elongated member, an inner elongated member positioned within and at least one intermediate layer positioned between the outer elongated member and the inner elongated member. At least one of the outer member, the inner member and the intermediate layer(s) contain channels at least partially filled with a pharmacologically active ingredient. In certain embodiments the channels form an open foam structure. In one embodiment, at least one of the outer member, inner member or the intermediate layer(s) include a silicone, a polyurethane or an elastomer.

The implantable medical device is a catheter, a urethral catheter, a catheter for suprapubic drainage, a catheter for nephrostomy drainage, a catheter for nasal pancreatic drainage or a nasal biliary drainage catheter.

In certain embodiments, the implantable medical device is configured to allow replenishment of the pharmacologically active ingredient after partial implantation of the device in a patient.

In one embodiment, the pharmacologically active ingredient comprises an alpha blocker. In another embodiment, the pharmacologically active ingredient comprises a calcium channel blocker.

In yet another embodiment, the pharmacologically active ingredient comprises a thrombin inhibitor, an antithrombogenic agent, or a mixture of a thrombin inhibitor or an antithrombogenic agent.

In another embodiment, the pharmacologically active ingredient is an antiproliferative agent, an anti-cancer chemotherapeutic agent, an antiviral agent, an antimicrobial agent, an analgesic, an anesthetic, an antiflammatory agent or an antiencrustation compound.

In another embodiment, the pharmacologically active ingredient is rifampin, minocycline, or a mixture of rifampin and minocycline.

In yet another embodiment, the pharmacologically active ingredient is urokinase, streptokinase, a tissue plasminogen activator, heparin, covalent heparin, hirudin, hirulog, argatroban, D-phenylalanyl-L-poly-L-arginyl chloromethyl ketone, an antiplatelet compound or a mixture thereof.

In another embodiment, the pharmacologically active ingredient comprises an antimicrobial. In yet another embodiment, the pharmacologically active ingredient is bupivacaine.

Another aspect of the present invention provides a method of preparing an implantable medical device. The method includes forming the implantable medical device from a material comprising at least one elastomer, forming channels in at least a portion of the elastomer and contacting at least a portion of the elastomer with a pharmacologically active ingredient under conditions wherein the pharmacologically active ingredient impregnates a least a portion of the channels.

Yet another aspect of the present invention provides a method of delivering a pharmacologically active ingredient to a patient. The method includes at least partially implanting within the patient a non-metallic medical device having an outer elongated member, an inner elongated member positioned within the outer elongated member and at least one intermediate layer positioned between the outer member and the inner member. At least one of the outer member, the inner member and the at least one intermediate layer includes channels containing a pharmacologically active ingredient. The medical device is present within the patient for a time period sufficient to allow at least a portion of the pharmacologically active ingredient to be delivered to the patient.

### Brief Description of the Drawings

FIG. 1 depicts a cross-sectioned end view of a preferred embodiment of the implantable medical device of the present invention;
FIG. 2 depicts a cross-sectioned end view of another preferred embodiment of the implantable medical device of the present invention;
FIG. 3 depicts a partial, sectioned side view of the implantable medical device of the present invention;
FIG. 4 depicts a plan view of a Foley catheter;
FIG. 5 depicts cross-sectional views of a ureteral stent;
FIG. 6 depicts a partial cross section view of a ureteral stent; and
Fig. 7 depicts a portion of an implantable medical device with channels for storing a pharmacologically active ingredient.

### Detailed Description

### Definitions

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention. All publications, patent applications, patents and other references mentioned herein are incorporated by reference in their entirety. The materials, methods, and examples disclosed herein are illustrative only and not intended to be limiting.

As used herein the terms "comprise(s)," "include(s)," "having," "has," "can," "contain(s)," and variants thereof, are intended to be openended transitional phrases, terms, or words that do not preclude the possibility of additional acts or structures. The present invention also contemplates other embodiments "comprising," "consisting of" and "consisting essentially of," the embodiments or elements presented herein, whether explicitly set forth or not.

The terms "about" or "substantially" used with reference to a quantity includes variations in the recited quantity that are equivalent to the quantity recited, such as an amount that is insubstantially different from a recited quantity for an intended purpose or function.

As used herein, the term "implantable" refers to an ability of a medical device to be positioned, partially or wholly, at a location within a body of a human or veterinary patient for any suitable period of time, such as within a body vessel. For example, the medical device may be implanted within an esophagus, trachea, colon, biliary tract, urinary tract, or vascular system of a patient. Furthermore, the terms "implantation" and "implanted" refer to the positioning of a medical device, partially or wholly, at a location within a body, such as within a body vessel. Implantable medical devices can be configured for transient placement within a body vessel during a medical intervention (e.g., minutes to hours), or to remain in a body vessel for a prolonged period of time after an implantation procedure (e.g., weeks or months or years). Implantable medical devices can include devices configured for bioabsorption within a body during a prolonged period of time.

The term "biocompatible" refers to a material that is substantially non-toxic in the in vivo environment of its intended use, and that is not substantially rejected by the patient's physiological system (i.e., is non-antigenic). This can be gauged by the ability of a material to pass the biocompatibility tests set forth in International Standards Organization (ISO) Standard No. 10993 and/or the U.S. Pharmacopeia (USP) 23 and/or the U.S. Food and Drug Administration (FDA) blue book memorandum No. G95-1, entitled "Use of International Standard ISO-10993, Biological Evaluation of Medical Devices Part-1: Evaluation and Testing." Typically, these tests measure a material's toxicity, infectivity, pyrogenicity, irritation potential, reactivity, hemolytic activity, carcinogenicity and/or immunogenicity. A biocompatible structure or material, when introduced into a majority of patients, will not cause an undesirably adverse, long-lived or escalating biological reaction or response. Such a response is distinguished from a mild, transient inflammation which typically accompanies surgery or implantation of foreign objects into a living organism.

As used herein, the phrase "controlled release" refers to the release of a material, such as a pharmacologically active ingredient, at a predetermined rate. A controlled release may be characterized by a drug elution profile, which shows the measured rate that the material is removed from a material-containing device in a given solvent environment as a function of time. A controlled release does not preclude an initial burst release associated with the deployment of the medical device. In some embodiments of the invention an initial burst, followed by a more gradual subsequent release, may be desirable. The release may be a gradient release in which the concentration of the material released varies over time or a steady state release in which the material is released in equal amounts over a certain period of time (with or without an initial burst release).

As used herein, the term "pharmacologically active ingredient" refers to any agent that produces an intended therapeutic effect on the body to treat or prevent conditions or diseases.

As used herein, a "mixture" refers to a combination of two or more ingredients in which each ingredient retains its own chemical identity and properties.

### Implantable Medical Devices Having Channels

One aspect of the present invention provides an implantable medical device having channels at least partially filled with a pharmacologically active ingredient. Particular medical devices especially suited for application of combination materials with channels and pharmacologically active ingredients according to this invention include stents, catheters, urinary catheters, ureteral catheters or stents, long term urinary devices, tissue bonding urinary devices, penile prostheses, vascular grafts, vascular catheter ports, wound drain tubes, hydrocephalus shunts, peritoneal catheters, pacemaker capsules, artificial urinary sphincters, small or temporary joint replacements, urinary dilators, heart valves and the like.

In one embodiment, the medical devices according to the present invention are preferably fixed in size, having a constant cross-section, rather than being expandable in one or more dimensions. Examples of such devices are a double-pigtail ureteral stent and a urinary Foley catheter. These medical devices may have some variability in their inner diameter or outer diameter, i.e., in the sense that no device has perfect dimensional stability, and also in the sense that some parts of the device may be larger than other parts. When a ureteral stent is implanted into a ureter, or when a Foley catheter is implanted into a bladder and a urethra, there may be some compression of the walls of the stent or catheter, leading to a minor "change" in the inner diameter or outer diameter of the device. However, except for the balloon of the Foley catheter, these devices are not "radially expandable." These devices are thus not similar to a vascular stent in which there is an intentional and desired change in the radial dimension so that the stent may be implanted and expanded to fulfill its intended purpose in a blood vessel. Implantable medical devices having a constant cross section include urinary catheters and ureteral stents, which have a majority portion with a constant cross section that does not change upon insertion.

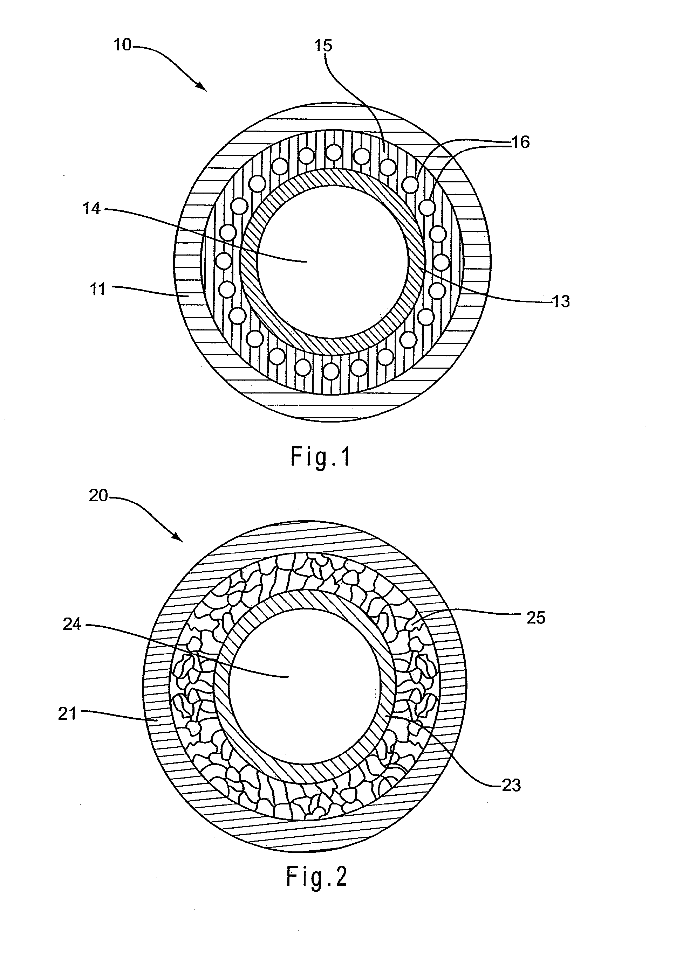

FIG. 1 depicts a cross-sectioned end view of a preferred illustrative embodiment of implantable medical device 10 such as a catheter having an outer elongated member 11 with a passage extending longitudinally therein. Alternatively, the outer elongated member can be simply a first layer 11 of material. Positioned concentrically and in the passage of outer elongated member 11 is inner elongated member 13 with passage 14 extending longitudinally therein. Again, alternatively, the inner elongated member can be simply a second layer 13 of material adjacent first layer 11. An intermediate layer 15 having channels 16 containing a pharmacologically active ingredient is positioned between and in communication with the outer and inner elongated members 11 and 13. Of course, members 11 and/or 13 can also include channels containing the same, or a different, pharmacologically active ingredient.

Again, with reference to FIG. 1, in one embodiment, the channels within intermediate layer 15 can include one pharmacologically active ingredient with a higher diffusion rate, whilst another pharmacologically active ingredient with a slower diffusion rate is nearer to the outer surface of the device, for example, within channels of outer elongated member 11. Such a configuration enables the pharmacologically active ingredients to reach the "outer surfaces" substantially simultaneously.

In one such embodiment, the pharmacologically active ingredient contained within channels of the intermediate region 15 includes a mixture of the antimicrobial drugs minocycline and rifampin. For example, the channels may include a 50:50 mixture by weight of minocycline and rifampin. Minocycline, which has a lower diffusion rate than rifampin, is also present within outer member 11.

In an alternative embodiment, the intermediate region 15 can comprise multiple layers formed between the inner and outer elongated members and one or more of such layers can include channels containing pharmacologically active ingredients. Devices eluting multiple pharmacologically active ingredients having differing diffusion rates are described more fully in U.S. publication number 2004/0068241 A1, published April 8, 2004, the contents of which are incorporated by reference.

In one embodiment, outer elongated member 11 is approximately .125" in diameter with a wall thickness of approximately .007". Inner elongated member 13 has an inner diameter of approximately .062" with a wall thickness of .007". The pharmacologically active ingredient comprising a 50:50 mixture by weight of rifampin and minocycline is positioned within channels 16 within intermediate region 15 between the inner and outer elongated members 13 and 11. As a result, the thickness of the intermediate layer 15 having channels 16 containing the pharmacologically active ingredient mixture is approximately .017". The overall wall thickness of the catheter is approximately .031".

In one embodiment, the material of the outer intermediate and inner members is a silicone material having a durometer in a range of 30 to 90 on the Shore A Hardness Scale. Such a silicone material is commercially available from the NU-SIL Corporation of Carpinteria, CA. Of course, as is disclosed below, the device of FIG. 1 may be manufactured from materials other than a silicone and many other pharmacologically active ingredients, other than rifampin and minocycline, may be included in the channels of such a device.

FIG. 2 depicts a second embodiment of implanted medical device 20 such as a catheter with outer elongated member 21 and inner elongated member 23 positioned in the passage of outer elongated member 21. An intermediate layer 25 of a base material, such as foam silicone, having a substantially open foam structure is positioned between outer and inner elongated members 21 and 23.

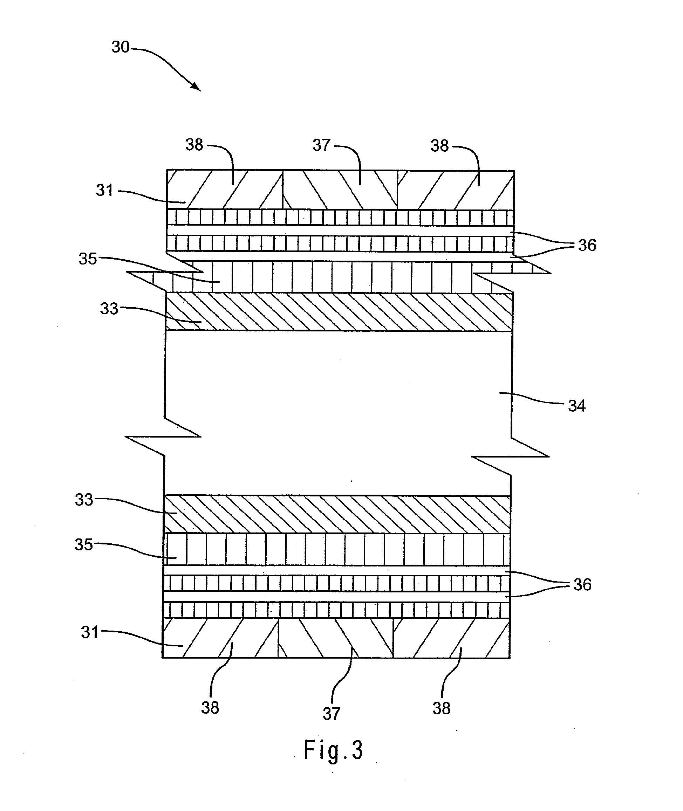

FIG. 3 depicts a partial, sectional side view of another embodiment of the present invention. Catheter 30 includes an outer elongated member 31 and an inner elongated member 33 separated by intermediate layer 35. Channels 36 run through at least a portion of the intermediate layer 35. In one embodiment, the channels run substantially the length of the intermediate layer 35 from a delivery point normally positioned outside the patient when the device is implanted to the required delivery site of the pharmacologically active ingredient. Outer elongated member 31 includes regions 38 that are substantially impermeable to the pharmacologically active ingredient and regions 37 that allow the pharmacologically active ingredient to elute from the outer surface of the device.

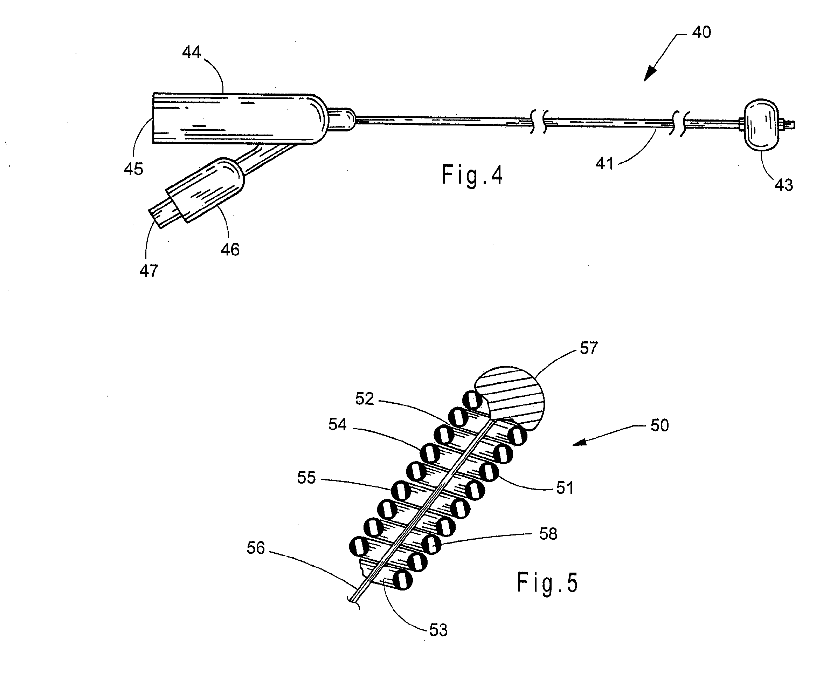

FIGS. 4 and 5 illustrate other medical devices applicable for use with the present invention. FIG. 4 depicts a Foley catheter 40, which includes elongated element 41 for draining urine from a urinary bladder of a patient. The Foley catheter has a constant cross section or diameter for most of the length of elongated element 41, except for a retention balloon 43. Balloon 43 is placed into the patient's bladder and is then inflated using fitting 46 and inflation lumen 47. Urine is drained from the patient through outlet 45 and outlet fitting 44, which may be used to connect to a container, such as a drainage bag. Elongated element 41 may be at least partly formed of a polymer or elastomer having channels.

FIG. 5 depicts a partial view of a ureteral stent. Ureteral stent 50 includes a length 51 of coiled wire 55 with spaces 52 between the coils so that urine can seep into central lumen 53. Coiled wire 55 is coated with one of more layers 54 having channels containing a pharmacologically active ingredient. For example, layers 54 may be formed from a polymer or elastomer. Stent 50 may have an internal rod 56 for securing end caps 57 to the stent. End caps 57 may also be secured to the end coils by soldering, welding, or brazing, or other joining technique.

Such Foley catheters or ureteral stents may contain channels that are impregnated, coated, or filled with a pharmacologically active ingredient. Many other medical devices may contain channels that are impregnated, coated or filled with a pharmacologically active ingredient, including but not limited to, a urethral catheter, a urethral stent, a prostatic stent, a biliary stent, a pancreatic stent, a catheter for suprapubic drainage, a catheter for nephrostomy drainage, a catheter for nasal pancreatic drainage, and a nasal biliary drainage catheter.

### Composition of the Implantable Medical Device

The implantable medical devices of the present invention are generally composed of one or more non-metallic materials. However, in certain embodiments, such devices may include a metallic base material at least partially coated with one of more layers of non-metallic materials. In one embodiment the devices comprise a polymer, preferably a biocompatible polymer. In various illustrative embodiments, the medical device includes cellulose acetate, cellulose nitrate, polyethylene teraphthalate, polyurethane, polyamide, polyester, polyorthoester, polyanhydride, polyether sulfone, polycarbonate, polypropylene, high molecular weight polyethylene, polytetrafluoroethylene, polylactic acid, polyglycolic acid, a polyanhydride, polycaprolactone, polyhydroxybutyrate valerate, silicone, poly(siloxane), or another biocompatible polymeric material, or mixtures or copolymers of these materials. In one preferred embodiment, the medical device includes a silicone or a polyurethane.

In other embodiments, the implantable medical devices comprise a rubber, elastomer, plastic, polyethylene, polyurethane, silicone, Gortex (polytetrafluoroethylene), DACRON^{®} (polyethylene terephthalate), TEFLON^{®} (a form of PTFE, polytetrafluoroethylene), latex, elastomer or DACRON^{®} sealed with gelatin, collagen or albumin.

### Formation of Channels

In a preferred embodiment, a pharmacologically active ingredient is present within channels formed within at least one layer of the device. For example, with reference to FIG. 1, channels can be formed in any of the intermediate layers, or the inner and/or outer members. The pharmacologically active ingredient may be placed within the channels before implantation, for example, during the manufacture of the device. Alternatively, the pharmacologically active ingredient can be supplied through the channels, for example, in a liquid form, after the device is positioned within the patient.

Channels may be formed in materials for stents, catheters, balloons, wire guides, cannulae, or other medical devices intended for implantation into a human or animal body. In one embodiment, the medical deice of the present invention is formed, at least partially, from a polymeric or elastomeric material, such as a polyurethane or a silicone. One way to form channels in such materials is to foam the material.

This is typically accomplished during formation, for instance, of silicone or urethane elastomers. The materials may be formulated to foam naturally, or they may be foamed by adding a blowing agent or a gas to the mixture. The foam thus formed may be open cell or closed cell foam. A large percentage of open cells is preferred to store a pharmacologically active ingredient in the medical device. One way of forming channels is to reticulate the base material, e.g., to physically process the material or foam to open the cells and form channels or a network of channels in the material. Alternatively, channels may be formed in the polymeric material during the molding or extrusion process.

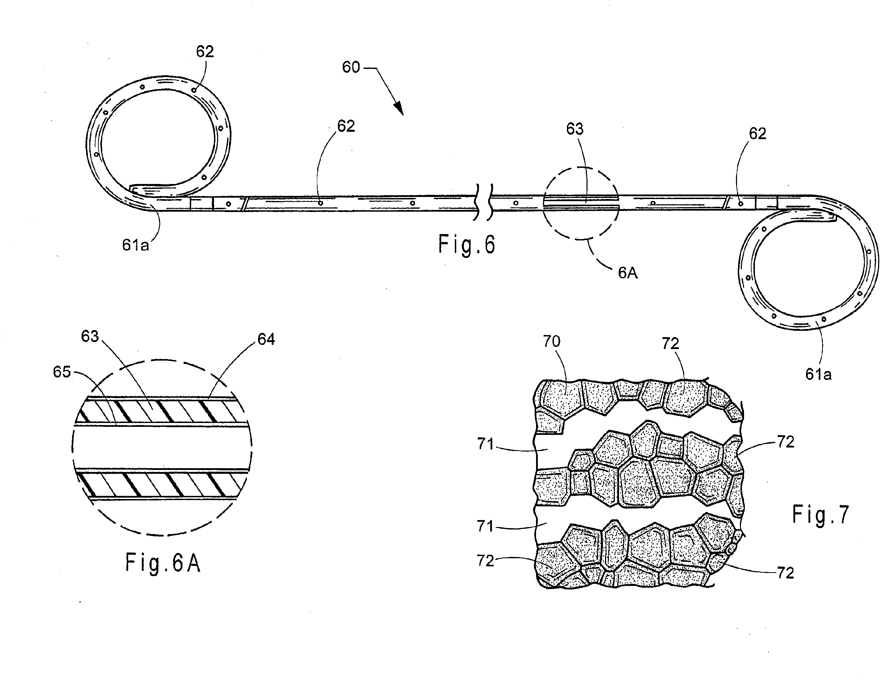

As mentioned above, channels may be formed through one or more of the inner member, outer member and the intermediate layers. FIG. 7 depicts an example of a structure with channels 71. A layer 70 of the device may have a cellular structure, with cells 72 and channels 71 between the cells. One or more pharmacologically active ingredients may reside in the channels for elution to the patient. The device may also be replenished by re-supply of the active ingredients, such as by injection of the ingredient into the channels.

Again, with reference to FIG. 1. In one embodiment, the intermediate layer 15 includes channels 16 containing a pharmacologically active ingredient. The thickness of the layer or layers and the density of channels within the layer may be designed for holding the desired amount of active ingredient and to obtain controlled release of the ingredient over a predetermined time. The thickness and material of outer layer 11 may also be used to obtain controlled release of pharmacologically active ingredient.

An additional advantage of medical devices made from multilayer materials with channels is that the active ingredients may be replenished or re-charged while the medical device remains implanted or partially implanted within the patient. Of course, such an embodiment requires that the channels are continuous from an entry point outside the body of the patient to the required site of treatment. Using a syringe, a nurse or medical professional may inject controlled additional dosages of the physiologically active ingredient or ingredients through channels in the intermediate or other layers as desired. Thus, the device could remain in place and continue its controlled diffusion of pharmacologically active ingredient into the patient.

In certain embodiments, it is desirable that only part of a device, such as a stent or a catheter, elutes a pharmacologically active ingredient. For instance, with a urethral or Foley catheter, only the portion of the catheter near or protruding through the urethral meatus may be required to elute an antimicrobial or anti-encrustation medicament. If a patient is being treated for cancer of the ureter, a ureteral stent eluting a pharmacologically active ingredient only in the central linear portion may be appropriate, rather than a device eluting such an ingredient from portions of the stent that will lie in the kidney or the bladder. Limiting the active ingredient eluting region of the implanted medical device to that portion where the ingredient is needed at least has the effect of reducing the amount of ingredient necessary for affective treatment of the patient. This may help reduce over-medicating of patients, will minimize any adverse drug reactions, and may also reduce any sensitization effects.

Medical devices of the present invention may be manufactured such that channels communicate with the surface of the device only within those regions where it is desired that the pharmacologically active ingredient be eluted from the surface. For example, only a portion of the length of a catheter may contain channels, such as an open foam structure. In such an embodiment, the pharmacologically active ingredient is loaded into, and elutes from, the region having channels. In another embodiment, foam channels are present throughout an intermediate region but this region is partially covered by a layer that is substantially non-permeable to the pharmacologically active ingredient. Such an embodiment allows for replenishment of the pharmacologically active ingredient while the device is implanted while permitting delivery of the active ingredient to selected regions of the patient's body.

For example, a ureteral stent which contains channels, for example, an open pore structure, only in the linear portion is depicted in partial cross-section in FIG. 6. Ureteral stent 60 is made from an elastomer, preferably urethane or silicone. The stent has a pigtail 61 a on either end, with orifices 62 for entrance and exit of urine or other fluids, such as irrigation fluid. In this instance, stent 60 only contains channels containing a pharmacologically active ingredient in the central, linear portion 63. Consequently, only the length of the ureter into which region 63 of stent 60 is placed will be treated with a pharmacologically active ingredient. For example, FIG. 6A depicts an expanded view of a portion of linear portion 63. Here, one or more of outer member 64, intermediate region 63 and inner member 65 can include channels having a pharmacologically active ingredient.

### Pharmacologically active ingredients

It is intended that the term pharmacologically active ingredient includes any material that is molecularly interactive with the fluids, cells, proteins or tissues of an animal including humans to augment the diagnosis, treatment or prevention of any physiologic or pathologic condition. It is further intended that this term includes therapeutic and diagnostic agents such as, for example, drugs, vaccines, hormones, steroids, proteins, previously described agents, complexing agents, salts, chemical compounds, polymers, and the like.

A vast range of drugs, medicaments and materials may be employed as a pharmacologically active ingredient within the channels of the device of the present invention so long as the selected ingredient can survive exposure to the conditions required to place the ingredient within the channels. Particularly useful in the practice of the present invention are materials which prevent or ameliorate abrupt closure and restenosis of blood vessels previously opened by stenting surgery or other procedures. Thrombolytics (which dissolve, break up or disperse thrombi) and antithrombogenics (which interfere with or prevent the formation of thrombi) are especially useful pharmacologically active ingredients when the implantable medical device is configured for insertion into the vascular system. Particularly preferred thrombolytics are urokinase, streptokinase and the tissue plasminogen activators. Particularly preferred antithrombogenics are heparin, hirudin, and the antiplatelets.

Urokinase is a plasminogen activating enzyme typically obtained from human kidney cell cultures. Urokinase catalyzes the conversion of plasminogen into the fibrinolytic plasmin, which breaks down fibrin thrombi.

Heparin is a mucopolysaccharide anticoagulant typically obtained from porcine intestinal mucosa or bovine lung. Heparin acts as a thrombin inhibitor by greatly enhancing the effects of the blood's endogenous antithrombin III. Thrombin, a potent enzyme in the coagulation cascade, is key in catalyzing the formation of fibrin. Therefore, by inhibiting thrombin, heparin inhibits the formation of fibrin thrombi. Alternatively, heparin may be covalently bound to the outer layer of the implantable medical device. Thus, heparin would form the outermost layer of the implantable medical device and would not be readily degraded enzymatically, and would remain active as a thrombin inhibitor.

Of course, pharmacologically active ingredients having other functions can also be successfully delivered by the device of the present invention. For example, an antiproliferative agent such as methotrexate will inhibit over-proliferation of smooth muscle cells and thus inhibit restenosis of the dilated segment of the blood vessel. The antiproliferative is desirably supplied for this purpose over a period of about four to six months. Additionally, localized delivery of an antiproliferative agent is also useful for the treatment of a variety of malignant conditions characterized by highly vascular growth. In such cases, the device of the present invention could be placed in the arterial supply of the tumor to provide a means of delivering a relatively high dose of the antiproliferative agent directly to the tumor.

A vasodilator such as a calcium channel blocker or a nitrate will suppress vasospasm, which is common following angioplasty procedures. Vasospasm occurs as a response to injury of a blood vessel, and the tendency toward vasospasm decreases as the vessel heals. Accordingly, the vasodilator is desirably supplied over a period of about two to three weeks. Of course, trauma from angioplasty is not the only vessel injury which can cause vasospasm, and the device may be introduced into vessels other than the coronary arteries, such as the aorta, carotid arteries, renal arteries, iliac arteries or peripheral arteries for the prevention of vasospasm in them.

A variety of other pharmacologically active ingredients are particularly suitable for use when the device is configured as something other than a coronary stent. For example, an anti-cancer chemotherapeutic agent can be delivered by the device to a localized tumor. More particularly, the device can be placed in an artery supplying blood to the tumor or elsewhere to deliver a relatively high and prolonged dose of the agent directly to the tumor, while limiting systemic exposure and toxicity. The agent may be a curative, a pre-operative debulker reducing the size of the tumor, or a palliative which eases the symptoms of the disease. It should be noted that the pharmacologically active ingredient in the present invention is delivered across the device, and not by passage from an outside source through any lumen defined in the device, such as through a catheter employed for conventional chemotherapy. The pharmacologically active ingredient of the present invention may, of course, be released from the device into any lumen defined in the device, or to tissue in contact with the device and that the lumen may carry some other agent to be delivered through it. For example, tamoxifen citrate, TAXOL^{®} (Paclitaxel) or derivatives thereof PROSCAR^{®} (Finasteride), HYTRIN^{®} (Terazosin)., or EULEXIN^{®} (flutamide) may be applied to the tissue-exposed surface of the device for delivery to a tumor located, for example in breast tissue or the prostate.

Dopamine or a dopamine agonist such as bromocriptine mesylate or pergolide mesylate is useful for the treatment of neurological disorders such as Parkinson's disease. The device could be placed in the vascular supply of the thalamic substantia nigra for this purpose, or elsewhere, localizing treatment in the thalamus.

A wide range of other pharmacologically active ingredients can be delivered by the device of the present invention. Accordingly, it is preferred that the pharmacologically active ingredient includes at least one of heparin, covalent heparin, or another thrombin inhibitor, hirudin, hirulog, argatroban, D-phenylalanyl-L-poly-L-arginyl chloromethyl ketone, or another antithrombogenic agent, or mixtures thereof; urokinase, streptokinase, a tissue plasminogen activator, or another thrombolytic agent, or mixtures thereof; a fibrinolytic agent; a vasospasm inhibitor; a calcium channel blocker, a nitrate, nitric oxide, a nitric oxide promoter or another vasodilator; HYTRIN^{®} or other antihypertensive agents; an antimicrobial agent or antibiotic; aspirin, ticlopidine, a glycoprotein IIb/IIIa inhibitor or another inhibitor of surface glycoprotein receptors, or another antiplatelet agent; colchicine or another antimitotic, or another microtubule inhibitor, dimethyl sulfoxide (DMSO), a retinoid or another antisecretory agent; cytochalasin or another actin inhibitor; or a remodeling inhibitor; deoxyribonucleic acid, an antisense nucleotide or another agent for molecular genetic intervention; methotrexate or another antimetabolite or antiproliferative agent; tamoxifen citrate, TAXOL^{®} (Paclitaxel) or derivatives thereof, or other anti-cancer chemotherapeutic agents; dexamethasone, dexamethasone sodium phosphate, dexamethasone acetate or another dexamethasone derivative, or another anti-inflammatory steroid or non-steroidal antiinflammatory agent; cyclosporin or another immunosuppressive agent; trapidal (a PDGF antagonist), angiopeptin (a growth hormone antagonist), angiogenin, a growth factor or an anti-growth factor antibody, or another growth factor antagonist; dopamine, bromocriptine mesylate, pergolide mesylate or another dopamine agonist; ⁶⁰Co (5.3 year half life), ¹⁹²Ir (73.8 days), ³²P (14.3 days), ¹¹¹In (68 hours), ⁹⁰Y (64 hours), ^{99m}Tc (6 hours) or another radiotherapeutic agent; iodine-containing compounds, barium-containing compounds, gold, tantalum, platinum, tungsten or another heavy metal functioning as a radiopaque agent; a peptide, a protein, an enzyme, an extracellular matrix component, a cellular component or another biologic agent; captopril, enalapril or another angiotensin converting enzyme (ACE) inhibitor; ascorbic acid, alpha tocopherol, superoxide dismutase, deferoxamine, a 21-aminosteroid (Iasaroid) or another free radical scavenger, iron chelator or antioxidant; a ¹⁴C-, ³H-, ¹³¹I-, ³²P- or ³⁶S-radiolabelled form or other radiolabelled form of any of the foregoing; estrogen or another sex hormone; AZT or other antipolymerases; acyclovir, famciclovir, rimantadine hydrochloride, ganciclovir sodium, NORVIR^{®} (ritonavir), CRIXIVAN^{®} (indinavir sulfate), or other antiviral agents; 5-aminolevulinic acid, meta-tetrahydroxyphenylchlorin, hexadecafluoro zinc phthalocyanine, tetramethyl hematoporphyrin, rhodamine 123 or other photodynamic therapy agents; an IgG2 Kappa antibody against Pseudomonas aeruginosa exotoxin A and reactive with A431 epidermoid carcinoma cells, monoclonal antibody against the noradrenergic enzyme dopamine beta-hydroxylase conjugated to saporin or other antibody targeted therapy agents; gene therapy agents; and enalapril and other prodrugs; PROSCAR^{®}, HYTRIN^{®} or other agents for treating benign prostatic hyperplasia (BHP) or a mixture of any of these; and various forms of small intestine submucosa (SIS).

Other pharmacologically active ingredients include additional drugs that are effective against urinary encrustation, in addition to heparin and other drugs listed above. These additional anti-encrustation drugs include triclosan, silver nitrate, ofloxacin, ciproflaxin, phosphorylcholine and trimethoprim. There are also additional antimicrobial drugs, including a penicillin, a cephalosporin, a carbepenem, a beta-lactam, an antibiotic, an aminoglycoside, a macrolide, a lincosamide, a glycopeptide, a tetracyline, a chloramphenicol, a quinolone, a fucidin, a sulfonamide, a trimethoprim, a rifamycin, an oxaline, a streptogramin, a lipopeptide, a ketolide, a polyene, an azole, and an echinocandin. Still other useful antimicrobial drugs with which an implantable medical device may be coated include alpha-terpineol, methylisothiazolone, cetylpyridinium chloride, chloroxyleneol, hexachlorophene, chlorhexidine and other cationic biguanides, methylene chloride, iodine and iodophores, triclosan, taurinamides, nitrofurantoin, methenamine, aldehydes, azylic acid, rifampycin, silver, benzyl peroxide, alcohols, and carboxylic acids and salts, and silver sulfadiazine. Also useful as antimicrobials are anthracyclines, such as doxorubicin or mitoxantrone, fluoropyrimidines such as 5-fluoroacil, and also podophylotoxins, such as etoposide. The salts and the derivatives of all of these are meant to be included as examples of antimicrobial drugs. Gendine, a mixture of chlorhexidine and Gentian Violet, is another useful antimicrobial drug.

Anticancer drugs may be useful to patients when placed into medical devices for at least partial insertion into a patient. These include docetaxel and its derivatives, fluoro-pyrimidines including 5-fluoroacil and its derivatives, hydroxyurea, mercaptopurine, cisplatin, anthracyclines including daunorubicin and doxorubicin and their derivatives, podophylotoxins including etoposide, and mitoxantrone and its derivatives, a folic acid antagonist other than methotrexate and its derivatives, a camptothecin, and a platinum complex.

Other pharmacologically active ingredients may also be used in various embodiments. Alpha-blockers are drugs that block receptors in arteries and smooth muscles. The action of the alpha-blocker relaxes blood vessels and leads to an increase in blood flow and a lower blood pressure, thus helping to control blood pressure or hypertension. In the bladder neck or urinary tract, alpha-blockers also relax the walls of the tract and enhance urinary flow, especially in persons suffering from prostatic hypertrophy (an enlarged prostate gland). Alpha-blocker drugs include doxazosin (CARDURA^{®}), alfuzosin (UROXATRAL^{®}), tamsulosin (FLOMAX^{®}), prazosin (MINIPRESS^{®}), and terazosin (HYTRIN^{®}).

Calcium channel blockers (CCBs) are drugs that block the entry of calcium into muscle cells. By blocking the entry of calcium, the contraction of the heart is decreased and the arteries are dilated, reducing pressure in the arteries and making blood flow easier. Calcium channel blockers that may be used in medical device embodiments include nisoldipine (SULAR^{®}), nifedipine (ADALAT^{®}, PROCARDIA^{®}), nicardipine (CARDENE^{®}), bepridil (VASCOR^{®}), isradipine (DYNACIRC^{®}), nimodipine (NIMOTOP^{®}), felodipine (PLENDIL^{®}), amlodipine (NORVASC^{®}), diltiazem (CARDIZEM^{®}), and verapamil (CALAN^{®}, ISOPTIN^{®}).

Other pharmacologically active ingredients that are useful in a human or mammalian body include analgesics and anesthetics. In general, an anesthetic works by interrupting the transmission of nerve impulses, and thus preventing the sensation of pain. Analgesics work on the peripheral and central nervous systems to reduce the perception of pain. Analgesics include Aspirin, naproxen, choline, diflunisal, and salsalate. Other analgesics include non-steroidal antiflammatory agents, such as naproxen, choline, diflunisal, salsalate, fenoprofen, flurbiprofen, ketoprofen, ibuprofen, oxaprozin, diclofenac, indomethacin, sulindac, acetoaminophen, tolmetin, meloxicam, piroxicam, meclofenamate, mefanimic acid, nabumetone, etodelac, keterolac, celecoxib, valdecoxib and rofecoxib, mixtures thereof, and derivatives thereof.

Other analgesics include opioids, synthetic drugs with narcotic properties, and narcotics such as alfentanil, buprenorphine, carfentanil, codeine, codeinone, dextropropoxyphene, dihydrocodeine, endorphin, fentanyl, hydrocodone, hydromorphone, methadone, morphine, morphinone, oxycodone, oxymorphone, pethidine, remifantanil, sulfentanil, thebaine, and tramadol, mixtures thereof, and derivatives thereof. Anesthetics which may be used as a pharmacologically active ingredient in medical devices for implantation include local anesthetics such as paracetamol, bupivacaine, prilocaine, levobupivicaine, dubucaine, ropivacaine, lidocaine, and novocaine.

Metals, especially heavy metals, and ionic compounds and salts of these metals, are known to be useful as antimicrobials even in very low amounts or concentrations. These ingredients are said to have an oligodynamic effect, and they are considered oligodynamic. The metals include silver, gold, zinc, copper, cerium, gallium, platinum, palladium, rhodium, iridium, ruthenium, osmium, zinc, bismuth, and others. Other metals with lower atomic weights also have an inhibiting or cidal effect on microorganisms in very low concentrations. These metals include aluminum, calcium, sodium, magnesium, potassium, manganese, and lithium, among others. For present purposes all these metals are oligodynamic metals, and their compounds and ionic ingredients are oligodynamic ingredients. The metals, their compounds and ions, e.g., zinc oxide, silver acetate, silver nitrate, silver chloride, silver iodide and many others, may inhibit the growth of microorganisms, such as bacteria, viruses, or fungi, or they may have cidal effects on microorganisms, such as bacteria, viruses, or fungi, in higher concentrations. Because many of these compounds and salts are soluble, they may easily be placed into solution, alone or with another physiologically active ingredient, and then absorbed into a medical device or adsorbed onto its surface.

The anionic portion of the compound or salt is desirably selected from among, but is not limited to, the oxide, acetate, ascorbate, benzoate, bitartrate, bromide, carbonate, chloride, citrate, folate, gluconate, iodate, iodide, lactate, laurate, oxalate, palmitate, perborate, phenosulfonate, phosphate, propionate, salicylate, stearate, succinate, sulfadiazine, sulfate, sulfide, sulfonate, tartrate, thiocyanate, thioglycolate, thiosulfate, and the like. Combinations of any of these may also be used.

Silver salts are particularly useful for their inhibiting and cidal effects on microorganisms, such as bacteria, viruses and fungi. Such salts include, but are not limited to, silver oxide, silver chloride, silver iodide, silver citrate, silver lactate, silver acetate, silver propionate, silver salicylate, silver bromide, silver ascorbate, silver laurel sulfate, silver phosphate, silver sulfate, silver benzoate, silver carbonate, silver sulfadiazine, silver gluconate, and combinations thereof.

Oligodynamic ingredients as defined above, including the metals, the salts, and other compounds, may advantageously be used in combination with other physiologically active ingredients. The salts and compounds may be particularly useful because, having different solubilities, the appropriate salts or compounds may be selected for the desired rate of release within the patient. The compound or compounds with the desired inhibiting or cidal effect and the desired release may be selected in coordination with another medicament or drug for the desired effect on the patient.

### Permeation with a Pharmacologically Active Ingredient

In one embodiment of the present invention, a pharmacologically active ingredient is impregnated into the channels of at least one layer of the implantable medical device. For example, a pharmacologically active ingredient can be impregnated into channels formed as an open pore structure. U.S. Patent No. 5,624,704, which is hereby incorporated by reference in its entirety, gives several examples of non-metallic medical devices intended for endoscopic or laparoscopic implantation into a patient. One or more pharmacologically active ingredients, such as analgesics or anesthetics, may be impregnated into channels of the devices of the present invention by using the ingredients, a solvent and a penetrating ingredient. For example, such a procedure can be used to impregnate a layer including an open pore structure. The solvent is preferably an organic solvent and the penetrating agent is an ingredient that enables the pharmacologically active ingredient to permeate the base material or layers of the device intended for implantation and to become deposited within the channels within the device.

The organic solvent may be any solvent that can be used to dissolve pharmacologically active ingredient, such as an antimicrobial agents. Such solvents include alcohols (e.g. methanol, ethanol), ketones (acetone, methylethylketone), ethers (tetrahydrofuran), aldehydes (formaldehyde), acetonitrile, acetic acid, methylene chloride and chloroform. The "penetrating agent" can be any compound that can be used to promote penetration of the ingredient into the material of the medical device. Examples of such compounds are esters (i.e. ethyl acetate, propyl acetate, butyl acetate, amyl acetate, and combinations thereof), ketones (i.e. acetone and methylethylketone), methylene chloride and chloroform. The "alkalinizing agent" can be an organic and inorganic base including sodium hydroxide, potassium hydroxide, ammonia in water (27% ammonium hydroxide), diethylamine and triethylamine. A "high ionic strength salt" may act both as an alkalinizing agent and as a penetrating agent. Such salts include sodium chloride, potassium chloride and ammonium acetate.

One embodiment of the present invention is a method for impregnating a non-metallic portion of a medical device with a pharmacologically active ingredient comprising the steps of forming a pharmacologically active ingredient of an effective concentration by dissolving the ingredient in an organic solvent, adding a penetrating agent to the composition and applying the ingredient to at least a portion of medical device under conditions where the pharmacologically active ingredient permeates the material of the medical device and is positioned within channels within the device.

Another embodiment of the present invention is a method for impregnating a non-metallic portion of a medical device with a pharmacologically active ingredient comprising the steps of forming a pharmacologically active ingredient of an effective concentration by dissolving the ingredient in an organic solvent, and applying the ingredient to at least a portion of medical device under conditions where the pharmacologically active ingredient permeates the material of the medical device and is positioned within channels within the device. In certain embodiments, the organic solvent is methanol or ethanol. In another embodiment, the pharmacologically active ingredient is bupivacaine.

In one embodiment, the step of dissolving a pharmacologically active ingredient may also include the step of adding an alkalinizing agent to the composition in order to enhance the reactivity of the material of the medical device. Further according to the preferred embodiment, the pharmacologically active ingredient is heated to a temperature between about 30°C and 70°C prior to applying the composition to the medical device to increase the adherence of the pharmacologically active ingredient to the medical device material. After the impregnated device is removed from the solution of a pharmacologically active ingredient and allowed to dry, the impregnated device is preferably rinsed with a liquid and milked to remove excess granular deposits and ensure uniform color of the impregnated device. The pharmacologically active ingredient may be applied to the medical device by dipping the implant into a solution of the dissolved ingredient for a period of between 15 and 120 minutes, and then removing the impregnated implant from the solution. Preferably, the device is dipped in the composition for a period of approximately 60 minutes.

The method of the present invention preferably comprises a single step of applying a pharmacologically active ingredient to the medical device. However, it is expected that several applications of the pharmacologically active ingredient, or other ingredients, can be applied to the device without affecting the adherence of the pharmacologically active ingredient to the device.

A preferred embodiment of the method for impregnating a catheter with a pharmacologically active ingredient that is an antimicrobial agent comprises the steps of (1) forming a pharmacologically active ingredient of an effective concentration to inhibit the growth of bacterial, viral, and or organisms, such as staphylococci, other gram-positive bacteria, gram-negative bacilli and Candida, by (a) dissolving a pharmacologically active ingredient in an organic solvent, (b) optionally adding a penetrating agent to the pharmacologically active ingredient and organic solvent composition, (c) optionally adding an alkalinizing agent to the composition to improve the reactivity of the material of the medical device; (2) heating the composition to a temperature of between about 30°C and 70°C to enhance the adherence of the pharmacologically active ingredient to the material of the medical device; (3) applying the pharmacologically active ingredient to the medical device, preferably by dipping the device in the composition for a period of about 60 minutes and under conditions where the pharmacologically active ingredient permeates the material of the medical device and becomes positioned within channels within the material; (4) removing the impregnated medical device from the pharmacologically active ingredient, and allowing it to dry; and (5) rinsing the impregnated medical device with a liquid and milking the impregnated medical device.

### Methods of Treating a Patient

Another aspect of the present invention provides for a method of administrating a pharmacologically active ingredient to treat a human or veterinary patient. In one embodiment, the method comprises implanting a medical device of the present invention into the body of the patient. Such a device contains channels including at least one of the pharmacologically active ingredients mentioned above.

In one embodiment, the implantable medical device is a urethral or ureteral catheter or stent, for example, a Foley catheter introduced into the patient's bladder via the urethra for the drainage of urine. In such an embodiment, an antimicrobial agent, such as chlorhexidine, is included to prevent or treat any infection associated with the implantation of the device. In another embodiment, an anesthetic agent, such as bupivacaine, may be incorporated into the device to reduce or eliminate pain associated with the placement of the device.

In another embodiment, where a catheter is introduced into the vascular system of a patient for long-term hemodialysis, an antithrombogenic agent may be included with the channels to prevent the formation of thrombi. In addition to these illustrative embodiments, other pharmacologically active ingredients, including, but not limited to, those described above may be included within the channels or the device to treat or prevent a wide range of physiologic or pathologic conditions. In certain embodiments, two or more pharmacologically active ingredients are incorporated into the device, either within the same region of the device or within separate regions, allowing for the treatment of multiple conditions. For example, one pharmacologically active ingredient is incorporated within the inner region and another pharmacologically active ingredient is incorporated within the outer region.

It is to be understood, that the above-described implantable medical device is merely an illustrative embodiment of the principles of this invention, and that other devices and methods for using them may be devised by those skilled in the art, without departing from the spirit and scope of the invention. It is to be understood that the invention is directed to embodiments both comprising and consisting of the disclosed parts. It is contemplated that only parts of the device can include the pharmacologically active ingredient. Furthermore, different parts of the device can include different pharmacologically active ingredients. It is also contemplated that different sides or regions of the same part of the device can include different pharmacologically active ingredients or layers. Accordingly, the invention should be limited only by the spirit and scope of the claims.

## Claims

1. A catheter comprising:
an outer elongated member;
an inner elongated member positioned within the outer elongated member; and
at least one intermediate layer positioned between the outer elongated member and the inner elongated member, wherein the at least one intermediate layer comprises an open foam structure containing a pharmacologically active ingredient,
wherein the outer elongated member comprises at least one region that is impermeable to the pharmacologically active ingredient and at least one region that allows pharmacologically active ingredient from the intermediate layer to elute from an outer surface of the device, and
wherein the foam extends from a delivery point positioned outside the patient along a length of the intermediate layer and enables the pharmacologically active ingredient to be replenished while the catheter is implanted.

2. The catheter of claim 1, wherein at least one of the outer elongated member, the inner elongated member and the at least one intermediate layer comprises a material selected from the group consisting of a silicone and a polyurethane.

3. The catheter of claim 1, wherein intermediate layer comprises a silicone or a polyurethane having a foam structure.

4. The catheter of claim 1, wherein at least one of the outer elongated member, the inner elongated member and the intermediate layer comprises an elastomer.

5. The catheter of claim 1, wherein the catheter is selected from the group consisting of a urethral catheter, a catheter for suprapubic drainage, a catheter for nephrostomy drainage, a catheter for nasal pancreatic drainage and a nasal biliary drainage catheter.

6. The catheter of claim 1, wherein the at least one pharmacologically active ingredient comprises an alpha blocker or a calcium channel blocker.

7. The catheter of claim 1, wherein the at least one pharmacologically active ingredient is selected from the group consisting of a thrombin inhibitor, an antithrombogenic agent, and a mixture of a thrombin inhibitor and an antithrombogenic agent.

8. The catheter of claim 1, wherein the at least one pharmacologically active ingredient is selected from the group consisting of an antiproliferative agent, an anti-cancer chemotherapeutic agent, an antiviral agent, an antimicrobial agent, an analgesic, an anesthetic, an antiflammatory agent and an antiencrustation compound.

9. The catheter of claim 1, wherein the at least one pharmacologically active ingredient is selected from the group consisting of rifampin, minocycline, and a mixture of rifampin and minocycline.

10. The catheter of claim 1, wherein the at least one pharmacologically active ingredient is selected from the group consisting of urokinase, streptokinase, a tissue plasminogen activator, heparin, covalent heparin, hirudin, hirulog, argatroban, D-phenylalanyl-L-poly-L-arginyl chloromethyl ketone, an antiplatelet compound, and mixtures thereof.

11. The catheter of claim 1, wherein the at least one pharmacologically active ingredient comprises an antimicrobial.

12. The catheter of claim 1, wherein the at least one pharmacologically active ingredient is bupivacaine.

13. The catheter of claim 1, wherein the catheter is a non-metallic device.

14. The catheter of claim 1, further comprising a delivery point positioned at an end of the catheter, wherein the delivery point allows delivery of the at least one pharmacologically active ingredient into the foam.

## Patentansprüche

1. Katheter, umfassend:
ein langgestrecktes Außenelement;
ein langgestrecktes Innenelement, das in dem langgestreckten Außenelement angeordnet ist; und
mindestens eine Zwischenschicht, die zwischen dem langgestreckten Außenelement und dem langgestreckten Innenelement angeordnet ist, worin die mindestens eine Zwischenschicht eine offene Schaumstruktur mit einem pharmakologischen Wirkstoff umfasst,
worin das langgestreckte Außenelement mindestens eine Region, die für den pharmakologischen Wirkstoff undurchlässig ist, und mindestens eine Region umfasst,
die Elution des pharmakologischen Wirkstoffs der Zwischenschicht von einer Außenseite der Vorrichtung gestattet, und
worin sich die Schaumstruktur von einem außerhalb des Patienten gelegenen Abgabepunkt entlang einer Länge der Zwischenschicht erstreckt und ein Nachfüllen des pharmakologischen Wirkstoffs, während der Katheter implantiert ist.

2. Katheter nach Anspruch 1, worin zumindest das langgestreckte Außenelement, das langgestreckte Innenelement und/oder die mindestens eine Zwischenschicht ein Material umfasst, das aus der aus einem Silikon und einem Polyurethan bestehenden Gruppe ausgewählt ist.

3. Katheter nach Anspruch 1, worin die Zwischenschicht ein Silikon oder ein Polyurethan mit einer Schaumstruktur umfasst.

4. Katheter nach Anspruch 1, worin zumindest das langgestreckte Außenelement, das langgestreckte Innenelement und/oder die Zwischenschicht ein Elastomer umfasst.

5. Katheter nach Anspruch 1, worin der Katheter aus der aus einem Harnröhrenkatheter, einem Katheter zur suprapubischen Drainage, einem Katheter zur Nephrostomie-Drainage, einem Katheter zur nasalen Pankreasdrainage und einem Katheter zur nasalen biliären Drainage bestehenden Gruppe ausgewählt ist.

6. Katheter nach Anspruch 1, worin der mindestens eine pharmakologische Wirkstoff einen Alphablocker oder einem Kalziumkanalblocker umfasst.

7. Katheter nach Anspruch 1, worin der mindestens eine pharmakologische Wirkstoff aus der aus einem Thrombin-Inhibitor, einem antithrombogenen Mittel, und einem Gemisch aus einem Thrombin-Inhibitor und einem antithrombogenen Mittel bestehenden Gruppe ausgewählt ist.

8. Katheter nach Anspruch 1, worin der mindestens eine pharmakologische Wirkstoff aus der aus einem proliferationshemmenden Mittel, einem chemotherapeutischen Antikrebsmittel, einem antiviralen Mittel, einem antimikrobiellen Mittel, einem Analgetikum, einem Narkosemittel, einem antiphlogistischen Mittel und einem Verkrustungen verhindernden Mittel bestehenden Gruppe ausgewählt ist.

9. Katheter nach Anspruch 1, worin der mindestens eine pharmakologische Wirkstoff aus der aus Rifampin, Minocyclin und einem Gemisch aus Rifampin und Minocyclin bestehenden Gruppe ausgewählt ist.

10. Katheter nach Anspruch 1, worin der mindestens eine pharmakologische Wirkstoff aus der aus Urokinase, Streptokinase, einem Gewebeplasminogenaktivator, Heparin, kovalentem Heparin, Hirudin, Hirulog, Argatroban, D-Phenylalanyl-L-poly-L-arginyl-chlormethylketon, einem Thrombozytenaggregationshemmer und Gemischen davon bestehenden Gruppe ausgewählt ist.

11. Katheter nach Anspruch 1, worin der mindestens eine pharmakologische Wirkstoff ein antimikrobielles Mittel umfasst.

12. Katheter nach Anspruch 1, worin der mindestens eine pharmakologische Wirkstoff Bupivacain ist.

13. Katheter nach Anspruch 1, worin der Katheter ein nicht-metallische Vorrichtung ist.

14. Katheter nach Anspruch 1, ferner einen an einem Ende des Katheters angeordneten Abgabepunkt umfassend, worin der Abgabepunkt die Abgabe des mindestens einen pharmakologischen Wirkstoffs in den Schaumstoff gestattet.

## Revendications

1. Cathéter, comprenant:
un élément allongé extérieur;
un élément allongé intérieur qui est positionné à l'intérieur de l'élément allongé extérieur; et
au moins une couche intermédiaire qui est positionnée entre l'élément allongé extérieur et l'élément allongé intérieur, dans lequel ladite au moins une couche intermédiaire comprend un structure alvéolaire ouverte contenant une substance pharmaceutiquement active,
dans lequel l'élément allongé extérieur comprend au moins une région qui est imperméable à la substance pharmaceutiquement active et au moins une région qui permet à la substance pharmaceutiquement active en provenance de la couche intermédiaire d'éluer à partir d'une surface extérieure du dispositif, et
dans lequel la mousse s'étend à partir d'un point de délivrance qui est positionné à l'extérieur du patient le long d'une longueur de la couche intermédiaire et qui permet le réapprovisionnement en substance pharmaceutiquement active lorsque le cathéter est implanté.

2. Cathéter selon la revendication 1, dans lequel au moins un parmi 1"élément allongé extérieur, l'élément allongé intérieur et ladite au moins une couche intermédiaire comprend un matériau qui est sélectionné dans le groupe comprenant un silicone et un polyuréthane.

3. Cathéter selon la revendication 1, dans lequel la couche intermédiaire comprend un silicone ou un polyuréthane présentant une structure alvéolaire.

4. Cathéter selon la revendication 1, dans lequel au moins un parmi l'élément allongé extérieur, l'élément allongé intérieur et la couche intermédiaire comprend un élastomère.

5. Cathéter selon la revendication 1, dans lequel le cathéter est sélectionné dans le groupe comprenant un cathéter urétral, un cathéter pour le drainage suspubien, un cathéter pour le drainage néphrostomique, un cathéter pour le drainage pancréatique nasal et un cathéter pour le drainage biliaire nasal.

6. Cathéter selon la revendication 1, dans lequel ladite au moins une substance pharmaceutiquement active comprend un alpha-bloquant ou un bloqueur du canal calcique.

7. Cathéter selon la revendication 1, dans lequel ladite au moins une substance pharmaceutiquement active est sélectionnée dans le groupe comprenant un inhibiteur de thrombine, un agent anti-thrombogénique et un mélange d'un inhibiteur de thrombine et d'un agent anti-thrombogénique.

8. Cathéter selon la revendication 1, dans lequel ladite au moins une substance pharmaceutiquement active est sélectionnée dans le groupe comprenant un agent antiprolifératif, un agent chimiothérapeutique anti-cancer, un agent antiviral, un agent antimicrobien, un analgésique, un anesthésique, un agent anti-inflammatoire et un composé anti-incrustation.

9. Cathéter selon la revendication 1, dans lequel ladite au moins une substance pharmaceutiquement active est sélectionnée dans le groupe comprenant la rifampicine, la minocycline et un mélange de rifampicine et de minocycline.

10. Cathéter selon la revendication 1, dans lequel ladite au moins une substance pharmaceutiquement active est sélectionnée dans le groupe comprenant l'urokinase, la streptokinase, un activateur tissulaire du plasminogène, l'héparine, l'héparine covalente, l'hirudine, l'hirulog, l'argatroban, la D-phénylalanyl-L-poly-L-arginyle chlorométhyle cétone, un composé antiplaquettaire ainsi que des mélanges de ceux-ci.

11. Cathéter selon la revendication 1, dans lequel ladite au moins une substance pharmaceutiquement active comprend un antimicrobien.

12. Cathéter selon la revendication 1, dans lequel ladite au moins une substance pharmaceutiquement active est la bupivacaïne.

13. Cathéter selon la revendication 1, dans lequel le cathéter est un dispositif non métallique.

14. Cathéter selon la revendication 1, comprenant en outre un point de délivrance qui est positionné à une extrémité du cathéter, dans lequel le point de délivrance permet la délivrance de ladite au moins une substance pharmaceutiquement active dans la mousse.
